# EUROPÄISCHE PATENTSCHRIFT

(11) **EP 0 966 254 B1**
(45) Veröffentlichungstag und Bekanntmachung des Hinweises auf die Patenterteilung: **17.04.2002**
(21) Anmeldenummer: 98904160.3
(22) Anmeldetag: 29.01.1998
(51) Int. Cl.: A61K 7/15

(54) **SCHÄUMENDE RASIERCREME**
FOAMING SHAVING CREAM
CREME A RASER MOUSSANTE

(30) Priorität: 07.02.1997 DE 19704635
(43) Veröffentlichungstag der Anmeldung: 29.12.1999
(73) Patentinhaber: Henkel Kommanditgesellschaft auf Aktien, 40589 Düsseldorf (DE)
(72) Erfinder: CORTEKAR, Hans-Wolfgang, D-42855 Remscheid (DE); SCHOLZ, Wolfhard, D-47829 Krefeld (DE)
(86) Internationale Anmeldenummer: EP9800472
(87) Internationale Veröffentlichungsnummer: WO9834586

(56) Entgegenhaltungen:
- EP-A- 0 285 574
- DE-A- 4 420 515
- US-A- 3 136 696

## Beschreibung

Die Erfindung betrifft eine schaumerzeugende Rasiercreme auf Basis synthetischer Tenside, die zur Abgabe aus einer Tube oder aus einem Dosierspender geeignet ist.

Als Rasiercreme im Sinne der vorliegenden Erfindung ist ein Hilfsmittel für die manuelle Naßrasur unter Verwendung einer Rasierklinge zu verstehen, das in Gegenwart von Wasser und unter mechanischer Einarbeitung von Luft mit Hilfe eines Pinsels oder Schwammes in einer Seifenschale oder direkt auf der Haut einen feinblasigen, stabilen Schaum entwickelt. Dieser Schaum soll vor der Rasur die behaarte Haut benetzen und gleitfähig machen und das Barthaar weich machen.

Darüber hinaus muß ein solches Produkt gute Benetzungseigenschaften aufweisen und darf die Haut nicht reizen.

Die dosierte Abgabe aus einer Quetschtube oder aus einem Druckspender, wie er für Zahnpasten oder für Senf gebräuchlich ist, bedingt eine gewisse cremeförmige Konsistenz.

Für den genannten Zweck sind bisher überwiegend Zubereitungen auf Basis von Fettsäure-Seife verwendet worden, da Seife die Anforderungen ein einen feinblasigen Schaum und an eine gel- oder cremeförmige Konsistenz recht gut erfüllt und darüber hinaus recht preiswert ist.

Seifen haben aber den Nachteil, daß sie gegen Wasserhärte empfindlich sind. Aus diesem Grunde enthielten solche Zusammensetzungen oft Zusätze von synthetischen Tensiden zur Dispergierung der Kalkseife. Auch sind Seifen stets alkalisch, was sich zwar günstig auf die Weichmachung des Barthaars auswirkt, aber bei empfindlichen Personen zu Hautreizungen führen kann.

Als Alternative wurden bisher meist nichtschäumende Rasiercremes auf Basis von Emulsionen langkettiger Fettalkohole, z.B. gemäß EP 285 574 A2 oder von Glycerin. Wasser und Carboxymethylcellulose gemäß WO 93/18740 A1 verwendet.

Es bestand daher die Aufgabe, eine Rasiercreme auf Basis von hautfreundlichen synthetischen, nicht härteempfindlichen Tensiden zu entwickeln, die sich aus einer Quetschtube, einer Quetschflasche oder einem Druckspender ohne Anwendung von Treibgasen entnehmen läßt, und die in Gegenwart von Wasser mit einem Pinsel oder einem Schwamm einen reichhaltigen, feinblasigen und stabilen Schaum bildet, der die Haut und das Barthaar rasch benetzt und für die Rasur geeignet macht.

Diese Aufgabe wurde erfindungsgemäß gelöst durch eine Rasiercreme auf Basis von synthetischen, anionischen und nichtionischen Tensiden, die
- 15 - 30 Gew.-%: in Wasser lösliche nichtionische Tenside
- 5 - 20 Gew.-%: in Wasser lösliche anionische Tenside
- 1 - 10 Gew.-%: in Wasser lösliche Polyole mit 2 - 10 C-Atomen und 2 - 7 Hydroxylgruppen sowie
- 40 Gew.-%: oder mehr Wasser enthalten.

Als wasserlöslich werden dabei solche nichtionische Tenside angesehen, die zu wenigstens 15 Gew.-% bei 20°C löslich sind bzw. solche anionischen Tenside, die zu wenigstens 5 Gew.-% in Wasser löslich sind.

Als wasserlösliche Polyole werden z.B. 1,2-Propylenglycol, Ethylenglycol, Glycerin, Erythrit, Sorbit, Mannit, Methylglucosid, Diglycerin, Triglycerin oder Pentaerythrit eingesetzt.

Bevorzugt sind als Polyole Glycerin, Sorbit oder Gemische davon, besonders bevorzugt sind Glycerin und Sorbit in einem Gewichtsverhältnis von 1 : 1 bis 3 : 1 und in einer Gesamtmenge von 1 bis 5 Gew.-% enthalten.

Als wasserlösliche nichtionische Tenside eignen sich insbesondere Alkylpolyglycolether, die eine lineare Alkylgruppe mit 12 - 22 C-Atomen und eine Polyethylenglycoletherkette mit 10 - 40 Glycolethergruppen aufweisen, gegebenenfalls im Gemisch mit anderen wasserlöslichen oder in Wasser dispergierbaren nichtionischen Tensiden. Solche weiteren nichtionischen Tenside können z.B. solche sein, die als hydrophile Gruppe eine Polyolgruppe, z.B. einen Sorbitanrest, einen Glycerin- oder Polyglycerinrest, einen Methylglucosidrest oder einen Polyglucosidrest enthalten.

Während die gesättigten Alkylpolyglycolether dazu beitragen, der Rasiercreme die erforderliche Konsistenz zu verleihen, tragen ungesättigte Alkenylpolyglycolether und nichtionische Tenside mit hydrophilen Polyolgruppierungen dazu bei, daß die Rasiercreme sich leichter aus der Tube ausdrücken und mit Wasser anschäumen läßt.

In einer bevorzugten Ausführung enthält die erfindungsgemäße Rasiercreme als nichtionische Tensidkomponente ein Gemisch aus gesättigten, linearen Fettalkoholpolyglycolethern und Fettsäure-Polyol-Ethylenoxid-Kondensationsprodukten. Als besonders geeignet zur Einstellung einer Konsistenz, die ein leichtes Ausdrücken aus der Tube erlaubt und dennoch einen formstabilen Produktstrang liefert, hat sich ein Gemisch aus einem Anlagerungsprodukt von 10 - 40 Mol Ethylenoxid an einen linearen, gesättigten Fettalkohol und einem Glycerin-Oxethylat-Fettsäuremonoester oder Fettsäuremonoglycerid-Oxethylat im Gewichtsverhältnis 1 : 3 bis 3 : 1 als nichtionisches Tensid erwiesen. Durch die Art und Menge dieser nichtionischen Tensidkomponenten läßt sich die Konsistenz der erfindungsgemäßen Rasiercreme in den gewünschten Bereich von 10 bis 30 Pa.s (bei 20°C) bringen.

Als wasserlösliches anionisches Tensid wird bevorzugt ein stark schäumendes Sulfatoder Sulfonat-Tensid, z.B. ein Fettalkohol-(C₁₂-C₁₆)-sulfat (in Form eines Alkanolammoniumsalzes), ein Fettalkohol-(C₁₂-C₁₆)-polyglycolethersulfat, bevorzugt in Form eines Alkali- oder Magnesiumsalzes, ein Sulfobemsteinsäure-mono-alkyl-(C₁₂-C₁₈)-ester-Salz, ein C₁₂-C₁₈-Acyloxyethersulfonat-Salz, ein C₁₂-C₁₈-Acyltaurid oder ein C₁₂-C₁₈-Fettsäuremonoglycerid-sulfat-Salz, bevorzugt in Form eines Alkali-, Ammonium- oder Alkanolammonium-Salzes eingesetzt.

Fettsäure-Seifen sollten möglichst nicht, allenfalls in Mengen von weniger als 1 Gew.-% enthalten sein.

Andere synthetische Tenside, z.B. Acylsarkoside oder Acylaminocarbonsäuren, acylierte Proteinhydrolysate, Ethercarbonsäuren der allgemeinen Formeln RO(CH₂CH₂O)ₙ-CH₂-COOH oder RCONH(CH₂CH₂O)ₙ-CH₂-COOH, in der R eine C₈-C₁₈-Alkylgruppe und n = 1 bis 6 ist oder deren Alkali- oder Ammoniumsalze können in untergeordneten Mengen von bis zu 5 Gew.-% in der Rasiercreme enthalten sein.

Als anionisches, schaumstarkes Tensid ist bevorzugt ein Fettalkoholpolyglycolethersulfat-Salz der Formel R¹O(C₂H₄O)ₓ OSO₃⁻ M⁺ enthalten, in der R¹ eine C₁₀-C₁₆-Alkylgruppe und x = 10 bis 18 ist, und M ein Natrium-, Kalium-, Ammonium- oder ein Mg⁺⁺/2-Ion ist.

Eine erfindungsgemäße Rasiercreme, die bezüglich Konsistenz. Anschäumungsvermögen sowie Feinblasigkeit und Stabilität des Schaums besonders optimiert ist, enthält
- 5 - 15 Gew.-%: eines Anlagerungsproduktes von 20 - 40 Mol Ethylenoxid an einen gesättigten, linearen C₁₆-C₁₈-Fettalkohol,
- 10 - 20 Gew.-%: eines Glycerinoxethylat-Monofettsäureesters aus einem Anlagerungsprodukt von 5 - 10 Mol Ethylenoxid an 1 Mol Glycerin und einer linearen, gesättigten C₁₂-C₁₈-Fettsäure,
- 10 - 20 Gew.-%: eines Alkyl-(C₁₂-C₁₆)-polyglycolethersulfat-Natriumsalzes mit 1 - 6 Glycolethergruppen,
- 1 - 5 Gew.-%: Glycerin, Sorbit oder eines Gemisches davon,
- 50 - 60 Gew.-%: Wasser und
übliche Hilfs- und Zusatzmittel in einer Menge von nicht mehr als 20 Gew.-%.

Als übliche Hilfs- und Zusatzmittel werden im Rahmen der vorliegenden Erfindung vor allem Duftstoffe, Farbstoffe, Pigmente und Perlglanzzusätze, andere nichtionische und anionische Tenside, Polyole und Emulgatoren sowie hautkosmetische und dermatologische Wirkstoffe, z.B. entzündungshemmende Wirkstoffe und Pflanzenextrakte, blutstillende oder antiseptische Komponenten, pH-Stellmittel bzw. Puffersubstanzen zur Einstellung eines pH-Wertes von 6 - 8, Komplexbildner oder rückfettende Komponenten empfohlen.

Als perlglanzgebende Zusätze eignen sich z.B. Dispersionen von Ethylenglycol-Distearat oder Diethylenglycol-Distearat in nichtionischen oder anionischen Tensidlösungen. Als rückfettende Ölkomponenten können z.B. Fettalkohole mit 12 - 22 C-Atomen oder verzweigte Alkohole mit 16 - 24 C-Atomen, Fettsäureester der Fettsäuretriglyceride, Paraffinöl, Silikonöl oder andere als kosmetische Ölkomponenten geeignete Stoffe, bevorzugt in emulgierter Form, in den erfindungsgemäßen Rasiercremes enthalten sein.

Als epithelisierender, die Heilung fördernder Wirkstoff kann z.B. Allantoin oder Harnstoff enthalten sein. Als kühlende und antiseptische Zusätze können z.B. Menthol oder Kampfer enthalten sein.

Die folgenden Beispiele sollen den Erfindungsgegenstand näher erläutern:

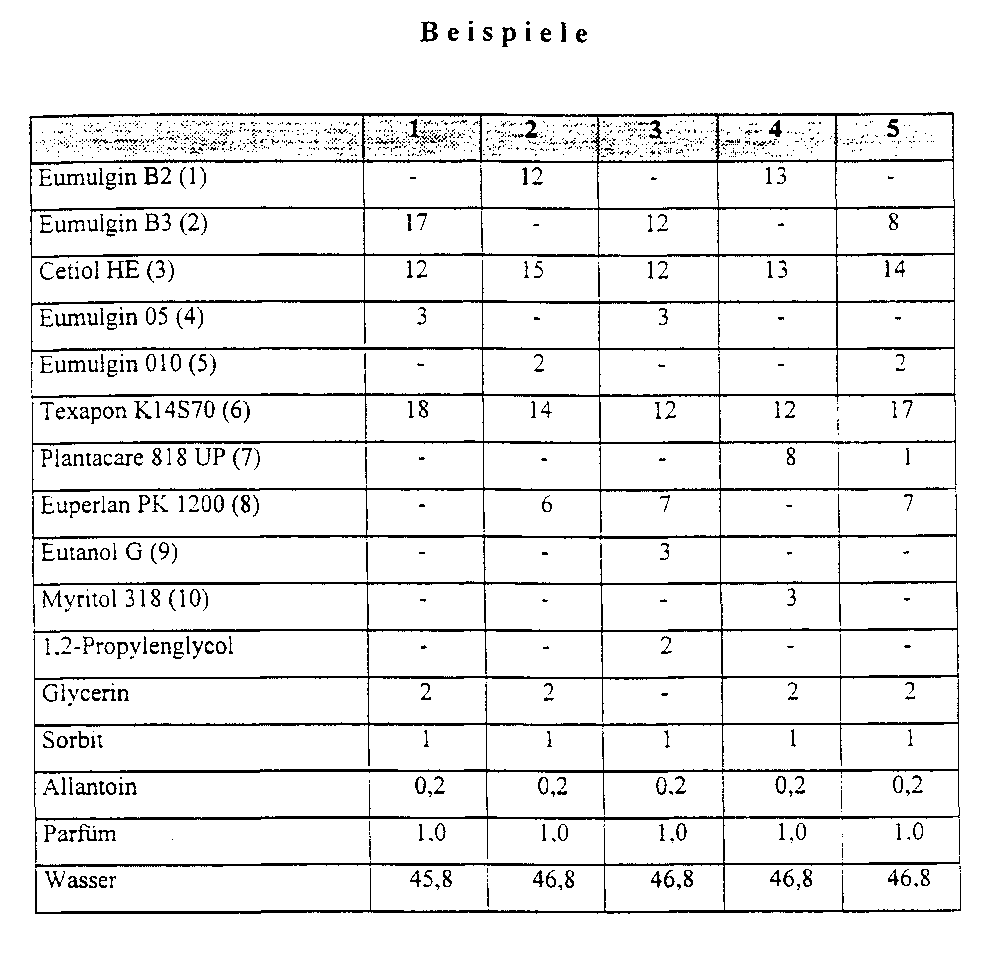

Die Viskosität bei 20°C (Haake Rotovisko, Meßsystem MV3) dieser Rezepturen liegt zwischen 20 und 30 Pa.s.

Es wurden die folgende Handelsprodukte eingesetzt:

| | |
|---|---|
| Eumulgin B2 | Cetyl-/Stearylalkohol + 20 Mol EO |
| Eumulgin B3 | Cetyl-/Stearylalkohol + 30 Mol EO |
| Cetiol HE | Glycerin + 7 EO-Kokosfettsäuremonoester |
| Eumulgin 05 | Cetyl-/Oleylalkohol + 5 Mol EO |
| Eumulgin 010 | Cetyl-/Oleylalkohol + 10 Mol EO |
| Texapon K14S70 | C_{12/14}-Fettalkohol + 3 Mol EO-sulfat, Natriumsalz, 70 %ig in H₂O |
| Plantacare 818 UP | C₈-C₁₄-Alkyl-poly-(1,4)-glucosid, 50 %ig in H₂O |
| Euperlan PK 1200 | Perlglanzkonzentrat (Alkylpolyglucosid, Ethylenglycolstearat, Glycerin) |
| Eutanol G | 2-Octyl-dodecanol |
| Myritol 318 | C₈-C₁₀-Fettsäure-triglycerid. |

## Patentansprüche

1. Rasiercreme auf Basis von synthetischen anionischen und nichtionischen Tensiden, **gekennzeichnet durch** einen Gehalt von
15 - 30 Gew.-% bei 20°C wasserlöslicher nichtionischer Tenside
5 - 20 Gew.-% bei 20°C wasserlöslicher anionischer Tenside
1 - 10 Gew.-% wasserlöslicher Polyole mit 2 - 10 C-Atomen und 2 - 7 Hydroxylgruppen
40 Gew.-% oder mehr Wasser.

2. Rasiercreme gemäß Anspruch 1, **dadurch gekennzeichnet, daß** Fettsäure-Seifen nicht oder in Mengen von weniger als 1 Gew.-% enthalten sind.

3. Rasiercreme nach Anspruch 1 oder 2, **dadurch gekennzeichnet, daß** als nichtionische Tenside ein Gemisch aus linearen Fettalkohol-Polyglycolethern und Fettsäure-Polyol-Ethylenoxid-Kondensationsprodukten enthalten ist.

4. Rasiercreme nach einem der Ansprüche 1 bis 3, **dadurch gekennzeichnet, daß** als Polyol Glycerin, Sorbit oder ein Gemisch davon enthalten ist.

5. Rasiercreme nach Anspruch 1 bis 4, **dadurch gekennzeichnet, daß** als nichtionischer Tensid ein Gemisch aus einem Anlagerungsprodukt von 10 - 40 Mol Ethylenoxid an einen linearen, gesättigten Fettalkohol mit 16 - 22 C-Atomen und einem Glycerinoxethylat-Fettsäuremonoester oder Fettsäuremonoglycerid-Oxethylat im Gewichtsverhältnis 1 : 3 bis 3 : 1 enthalten ist.

6. Rasiercreme nach einem der Ansprüche 1 bis 5, **dadurch gekennzeichnet, daß** als anionisches Tensid ein Fettalkoholpolyglycolethersulfat-Salz der allgemeinen Formel
R¹ - O (C₂H₄O)ₓ - OSO₃⁽⁻⁾M⁺
enthalten ist, in der R¹ eine C₁₀-C₁₆-Alkylgruppe, x = 1 bis 10 und M ein Natrium-, Kalium-, Ammonium- oder Mg/2-Ion ist.

7. Rasiercreme nach einem der Ansprüche 1 bis 6, enthaltend
5 - 15 Gew.-% eines Anlagerungsproduktes von 20 - 40 Mol Ethylenoxid an einen gesättigten, linearen C₁₆-C₁₈-Fettalkohol,
10 - 20 Gew.-% eines Glycerinoxethylat-Monofettsäureesters aus einem Anlagerungsprodukt von 5 - 10 Mol Ethylenoxid an 1 Mol Glycerin und einer linearen, gesättigten C₁₂-C₁₈-Fettsäure,
10 - 20 Gew.-% eines Alkyl-(C₁₂-C₁₆)-polyglycolethersulfat-Natriumsalzes mit 1 - 6 Glycolethergruppen,
1 - 5 Gew.-% Glycerin, Sorbit oder eines Gemisches davon, und
50 - 60 Gew.-% Wasser
sowie übliche Hilfs- und Zusatzmittel in einer Menge von nicht mehr als 20 Gew.-%.

## Claims

1. A shaving cream based on synthetic anionic and nonionic surfactants, **characterized in that** it contains
15 to 30% by weight of nonionic surfactants soluble in water at 20°C,
5 to 20% by weight of anionic surfactants soluble in water at 20°C,
1 to 10% by weight of water-soluble polyols containing 2 to 10 carbon atoms and 2 to 7 hydroxyl groups,
40% by weight or more of water.

2. A shaving cream as claimed in claim 1, **characterized in that** fatty acid soaps are present in quantities of less than 1% by weight, if at all.

3. A shaving cream as claimed in claim 1 or 2, **characterized in that** it contains a mixture of linear fatty alcohol polyglycol ethers and fatty acid/polyol/ethylene oxide condensates as nonionic surfactants.

4. A shaving cream as claimed in any of claims 1 to 3, **characterized in that** it contains glycerol, sorbitol or a mixture thereof as the polyol.

5. A shaving cream as claimed in claims 1 to 4, **characterized in that** it contains a mixture of an adduct of 10 to 40 moles of ethylene oxide with a linear saturated C₁₆₋₂₂ fatty alcohol and a glycerol ethoxylate fatty acid monoester or fatty acid monoglyceride ethoxylate in a ratio by weight of 1:3 to 3:1 as the nonionic surfactant.

6. A shaving cream as claimed in any of claims 1 to 5, **characterized in that** it contains a fatty alcohol polyglycol ether sulfate salt corresponding to the general formula:
R¹ - O(C₂H₄O)ₓ - OSO₃⁽⁻⁾M⁺
where R¹ is a C₁₀₋₁₆ alkyl group, x = 1 to 10 and M is a sodium, potassium, ammonium or Mg/2 ion, as the anionic surfactant.

7. A shaving cream as claimed in any of claims 1 to 6, **characterized in that** it contains
5 to 15% by weight of an adduct of 20 to 40 moles of ethylene oxide with a saturated linear C₁₆₋₁₈ fatty alcohol,
10 to 20% by weight of a glycerol ethoxylate monofatty acid ester of an adduct of 5 to 10 moles of ethylene oxide with 1 mole of glycerol and a linear saturated C₁₂₋₁₈ fatty acid,
10 to 20% by weight of an alkyl (C₁₂₋₁₆) polyglycol ether sulfate sodium salt containing 1 to 6 glycol ether groups,
1 to 5% by weight of glycerol, sorbitol or a mixture thereof,
50 to 60% by weight of water and
typical auxiliaries and additives in a quantity of not more than 20% by weight.

## Revendications

1. Crème à raser à base d'agents tensioactifs anioniques et non ioniques,
**caractérisée en ce qu'**
elle contient :
- de 15 à 30% en poids d'agents tensioactifs non ioniques solubles dans l'eau,
- de 5 à 20 % en poids d'agents tensloactifs anioniques solubles dans l'eau,
- de 1 à 10 % en poids de polyols solubles dans l'eau, ayant de 2 à 10 atomes de carbone et de 2 à 7 groupes hydroxyle, ainsi que
- 40 % en poids ou davantage d'eau.

2. Crème à raser conformément à la revendication 1,
**caractérisée en ce que**
des savons d'acide gras ne sont pas contenus ou sont contenus en quantités de moins de 1 % en poids.

3. Crème à raser selon la revendication 1 ou 2,
**caractérisée en ce que**
comme agents tensioactifs non ioniques, un mélange à base d'éthers d'alcool gras et de polyglycol et de produits de condensation d'acide gras-polyol-oxyde d'êthylène est contenu.

4. Crème à raser selon l'une des revendications 1 à 3,
**caractérisée en ce que**
comme polyol du glycérol, du sorbitol ou un mélange de ceux-ci, est contenu.

5. Crème à raser selon la revendication 1 à 4.
**caractérisée en ce que**
comme agent tensioactif non ionique, un mélange à base d'un produit d'addition de 10 à 40 mol d'oxyde d'éthylène sur un alcool gras linéaire. saturé ayant de 16 à 22 atomes de carbone et d'un monoester d'acide gras d'oxoéthylate de glycérol ou un oxoéthylate de monoglycéride d'acide gras dans un rapport en poids de 1 : 3 à 3 : 1 ; est contenu.

6. Crème à raser selon l'une des revendications 1 à 5,
**caractérisée en ce que**
comme agent tensioactif anionique un sel de sulfate d'éther d'alcool gras et de polyglycol de formule générale : R¹O(C₂H₄O)ₓ OSO₃⁻M⁺ est contenu dans laquelle R¹ est un groupe alkyle en C₁₀-C₁₆ et x = 1 à 10 et M est un ion sodium, potassium, ammonium ou un ion Mg⁺⁺/2.

7. Crème à raser selon l'une des revendications 1 à 6,
contenant :
- 5 à 15 % en poids d'un produit d'addition de 20 à 40 mol d'oxyde d'éthylène sur un alcool gras en C₁₆-C₁₈ saturé, linéaire,
- de 10 à 20 % d'un monoester d'acide gras d'oxoéthylate de glycérol à partir d'un produit d'addition de 5 à 10 mol d'oxyde d'éthylène sur 1 mol de glycérol et d'un acide gras en C₁₂-C₁₈ linéaire, saturé,
- de 10 à 20 % en poids d'un sel de sodium de sulfate d'éther d'alkyle en C₁₂-C₁₆ et de polyglycol ayant de 1 à 6 groupes d'éther de glycol,
- de 1 à 5 % en poids de glycérol, de sorbitol ou d'un mélange de ceux-ci,
- de 50 à 60 % en poids d'eau, et
- des adjuvants et des additifs usuels en une quantité qui n'est pas supérieure à 20 % en poids.
